# EUROPÄISCHE PATENTSCHRIFT

(11) **EP 1 294 907 B1**
(45) Veröffentlichungstag und Bekanntmachung des Hinweises auf die Patenterteilung: **29.10.2008**
(21) Anmeldenummer: 01955324.7
(22) Anmeldetag: 28.06.2001
(51) Int. Cl.: C12N 15/63, C12N 15/82, C12N 15/10

(54) **BINÄRVEKTOREN ZUR VERBESSERTEN TRANSFORMATION VON PFLANZLICHEN SYSTEMEN**
BINARY VECTORS FOR IMPROVED TRANSFORMATION OF PLANT SYSTEMS
VECTEURS BINAIRES POUR TRANSFORMATION AMELIOREE DE SYSTEMES VEGETAUX

(30) Priorität: 28.06.2000 EP 00113631
(43) Veröffentlichungstag der Anmeldung: 26.03.2003
(73) Patentinhaber: Sungene GmbH & Co. KGaA, 06468 Gatersleben (DE)
(72) Erfinder: HEIM, Ute, 06466 Gatersleben (DE); HERBERS, Karin, 06484 Quedlinburg (DE); KUNZE, Irene, 06466 Gatersleben (DE)
(74) Vertreter: Fitzner, Uwe
(86) Internationale Anmeldenummer: PCT/EP2001/007359
(87) Internationale Veröffentlichungsnummer: WO 2002/000900

(56) Entgegenhaltungen:
- EP-A- 0 265 556
- EP-A- 1 136 560
- WO-A-01/44482
- WO-A-99/01563
- XIANG C ET AL: "A mini binary vector series for plant transformation" PLANT MOLECULAR BIOLOGY, Bd. 40, Nr. 4, Juli 1999 (1999-07), Seiten 711-717, XP002143486 ISSN: 0167-4412
- JEONG H.L. ET AL.: "Construction of Small Binary Vectors for Argobacterium-Mediated Transformation of Plants" JOURNAL OF PLANT BIOLOGY, Bd. 42, Nr. 4, Dezember 1999 (1999-12), Seiten 317-320, XP008002458
- ZAMBRYSKI P. ET AL.: "TUMOR INDUCTION BY AGROBACTERIUM TUMEFACIENS: ANALYSIS OF THE BOUNDARIES OF T-DNA" JOURNAL OF MOLECULAR AND APPLIED GENETICS, Bd. 1, Nr. 4, 1982, Seiten 361-370, XP000926066 in der Anmeldung erwähnt
- ZUPAN J R ET AL: "TRANSFER OF T-DNA FROM AGROBACTERIUM TO THE PLANT CELL" PLANT PHYSIOLOGY, Bd. 107, Nr. 4, 1. April 1995 (1995-04-01), Seiten 1041-1047, XP002024243 ISSN: 0032-0889
- BROWN T.A.: "Gentechnologie für Einsteiger" SPEKTRUM AKADEMISCHER VERLAG * Seite 346 *

### SEQUENCE LISTING

<110> SunGene GmbH & Co. KGaA
<120> Binärvektoren zur verbesserten Transformation von pflanzlichen Systemen
<130> BASF/NAE 0146/00 PCT
<140>
   <141>
<160> 2
<170> PatentIn Ver. 2.1
<210> 1
   <211> 113
   <212> DNA
   <213> Artificial Sequence
<220>
   <223> Description of Artificial Sequence:additional Border Sequence aBS
<400> 1
   cagctgcttg gtaataattg tcattagatt gtttttatgc atagatgcac tcgaaatcag 60
   ccaattttag acaagtatca aacggatgtt aattcagtac attaaagacg tcc 113
<210> 2
   <211> 33
   <212> DNA
   <213> Artificial Sequence
<220>
   <223> Description of Artificial Sequence:part of
   additional Border Sequence aBS
<400> 2
   tgcatagatg cactcgaaat cagccaattt tag 33

## Beschreibung

Die vorliegende Erfindung betrifft ein System neuartiger Binärvektoren dadurch gekennzeichnet, daß sie trotz ihrer außergewöhnlich geringen Größe alle für den Transfer in die Agrobakterien und in die Pflanze erforderlichen Elemente enthalten.

Die Transformation von Pflanzen mit Hilfe des Agrobakterien-vermittelten Gentransfers ist in der Literatur beschrieben. Die auf den Ti-Plasmiden der Agrobakterien basierenden Vektoren erlauben die Transformation eines weiten Bereiches von Pflanzenspezies, indem sie ein natürliches bakterielles System zur Einführung von DNA in das nukleare Genom von Pflanzen nutzen. Als Teil dieses hochentwickelten Parasitismus transferiert das Agrobakterium einen definierten Teil seiner DNA, die T-DNA, die durch 25 bp lange Repeats, auch als linke und rechte Border bezeichnet, begrenzt ist, in die chromosomale DNA der Pflanzen (Zupan et al., The Plant Journal 23(1), 11-28. 2000).. Die kombinierte Aktion sogenannter vir Gene, die sich auf den ursprünglichen Ti-Plasmiden befinden, ermöglichen diesen DNA Transfer der T-DNA. *Agrobakterium* vermittelter Gentransfer nutzt die Vorteile dieses natürlichen pflanzlichen Transformationssystem. Hierzu wurden sogenannte Binärvektoren konstruiert, die einen mehr oder weniger effektiven Transfer von Nutzgenen oder Genkonstrukten erlauben. Frühere Untersuchungen ließen keine Gesetzmäßigkeiten hinsichtlich des Ortes des Einbaus der Fremd-DNA in das Genom der Pflanzen DNA erkennen. Gezeigt wurde, daß die durch Repeats (Left und Right Border) begrenzte T-DNA des Binärvektors relativ exakt übertragen wird (Tinland et al, Trends in Plant Science 1 (6), 178-184. 1996.). Wesentliche funktionelle Teile des Binärvektors sind die T-DNA mit den Borderrepeats, sowie die prokaryontischen Vektorsequenzen mit den Replikationsursprüngen (ori) für die Vermehrung und Erhaltung in den Bakterien (E. *coli* und Agrobakterium).

Die bisher beschriebenen Binärvektoren basieren auf den "broad host range" Plasmiden wie pRK252 (Bevan et al., Nucl. Acid Res. 12, 8711-8720, 1984) und pTJS75 (Watson et al., The EMBO Journal 4, no.2, 277-284, 1985), die ein breites Wirtsspektrum besitzen und von dem P-Typ Plasmid RK2 abgeleitet sind. Sie weisen allerdings den Nachteil auf, daß sie unter nicht selektiven Bedingungen instabil sind (Ditta et al, Plasmid 13, 149-153, 1985 ). Die WO 99/01563 beschreibt einen Vektor zur Transformation von Pflanzen enthaltend eine T-DNA mit flankierenden Border-Sequenzen und enthaltend eine außerhalb der T-DNA-liegende DNA-Sequenz (Gen kodierend für ein Toxin). Diese verhindert, dass sich transformierte Pflanzen entwickeln, welche andere Vektor-Sequenzen besitzen als die T-DNA. Eine große Gruppe der verwendeten Binärvektoren leitet sich vom pBIN19 (Bevan et al., Nucl. Acid Res. 12, 8711-8720, 1984) ab, der neben der Border des Ti-Plasmids pTiT37, den in Agrobakterien aktiven Replikationsursprung RK2 mit den relevanten cis Elementen oriv und ori_{T} sowie den ColE1 Ursprung des pBR322 enthält. Hajdukiewicz et al. entwickelte einen neuen Binärvektor (pPZP), der kleiner und effizienter als die bisher üblichen war (Hajdukiewicz et al, Plant Molecular Biology 25, 989-994, 1994). Anstatt des Replikationsursprungs RK2, besitzt der dort beschriebene Vektor pPZP den Replikationsursprung des Pseudomonas Plasmids pVS1, welcher die typische Organisation eines vollständigen Partitionslocus und damit Faktoren für die stabile Vererbung in den Agrobakterien über Generationen aufweist (Itoh et al., Plasmid 11, 206-220, 1984.). Das pVS1-Segment bewirkt durch sein vollständiges Partitionssystem die genetische Stabilität über Generationen hinweg ohne Selektionsdruck. Diese Eigenschaft, die die auf dem RK2 basierenden binären Vektoren nicht haben, scheint für eine höhere Transformationsrate beim Raps essentiell zu sein.

Frühere Analysen der für den Transfer notwendigen Sequenzen belegten, daß nur zwei cis- agierende Elemente essentiell sind. Diese zwei 25bp langen direkten nicht ganz identischen Repeats (sog. Borders) flankieren die T-DNA. Jede zwischen diesen Bordern befindliche DNA wird gerichtet transferiert (Wang et al., Cell 38, 455-462. 1984.). Während die rechte Border immer notwendig ist und sehr exakt geschnitten wird, verhindern Veränderungen an der linken Border nicht zwingend den DNA Transfer. Das linke Ende der transferierten DNA ist variabler. Aus diesem Grund werden neben der T-DNA mit den gewünschten transgenen Nukleotidsequenzen auch unerwünschte Vektor-Sequenzen, beispielsweise bakterielle Resistenzgene, in das Genom der Pflanzen übertragen (Hanson et al., The Plant Journal 19(6), 727-734. 1999, Kononov et al., The Plant Journal 11, 945-957. 1997, Martineau et al., The Plant Cell 6, 1032-1033. 1994, Ramanathan et al.,Plant MoLBiol. 28, 1149-1154. 1995). Dies ist jedoch insbesondere aus Sicherheitsgründen bei einer Anwendung dieser Pflanzen in der Agrarwirtschaft unerwünscht. Daher erfordern die bisher bekannten Systeme aufwendige molekulare Analysen der erzeugten transformierten Pflanzen, sofern sie für eine Nutzung in der Landwirtschaft freigegeben werden sollen.

Ein weiterer Nachteil existierender Binärvektoren ist die schlecht zu handhabende Größe und die niedrige Kopienzahl, beispielsweise des pBIN19 von 12 kb (Bevan et al., Nucl. Acid Res. 12, 8711-8720, 1984) oder des pGA482 von 13 kb (An et al., The EMBO Journal 4, no.2, 277-284. 1985). Die niedrige Kopienzahl führt zu niedrigen DNA Ausbeuten der Plasmide und erschwert die Klonierungen. Instabile Replikationsursprünge können zu einem variablen Verlust der Plasmide während der Vermehrung führen. Der Vektor pGreen mit einer Größe von 4,6 kb ist zwar sehr klein, ihm fehlen aber die Elemente zur stabilen Vermehrung in Agrobakterien, so daß man ihn nur zusammen mit speziellen Agrobakterienstämmen nutzen kann (Helles et al., Plant Molecular Biology 42, 819-832. 2000).
Vielfach fehlen auch eine ausreichende Zahl von Restriktionsenzymen zur Klonierung der gewünschten Expressionskassetten oder die Vektoren lassen nur die Verwendung weniger Selektionsmarker zu. Desweiteren fehlen den bisherigen Binärvektoren die Möglichkeit Selektionsmarker aus den transgenen Linien wieder zu entfernen. Einfache Sequenzanalysen mit Standardprimern sind nicht möglich, da Erkennungsstellen für kommerziell übliche Sequenzprimer nicht auf den Binärvektoren vorhanden sind. Unikale Restriktionsorte, die zusätzlich zu Multiklonierungsorten auf der T-DNA oder im Vektor liegen und eine modulare Handhabung ermöglichen, fehlen ebenso wie Restriktionsorte, die ein ,Genestacking' erlauben.
Aufgabe der vorliegenden Erfindung ist es somit, ein System zur Verfügung zu stellen, das die zuvor genannten Nachteile nicht mehr aufweist.

Diese Aufgabe wird erfindungsgemäß durch ein neues System von Binärvektoren gelöst, die sich durch ihre Komplexität und Modularität und unter anderem durch eine zusätzliche Border -Sequenz auszeichnen. Das Vorhandensein bestimmter von der Multiklonierungsstelle unabhängiger unikaler Restriktionsorte in der T-DNA, aber auch im Vektor, die T-DNA flankierend, eröffnet die Möglichkeit der Insertion beliebiger Module. Die Kombination der einzelnen Elemente ist neu.

Hierbei ist unter einem Binärvektor erfindungsgemäß ein Vektor zu verstehen, der sowohl in *E*. *coli* als auch in Agrobakterien vermehrt werden kann und die zur Übertragung in ein pflanzliches System erforderlichen Elemente enthält.

Gegenstand der Erfindung ist auch ein Verfahren zur Herstellung von Binärvektoren welche die oben beschriebene Aufgabe erfüllen. Vorzugsweise wird ein erfindungsgemäßer Binärvektor in 3 Schritten hergestellt:

Im ersten Schritt wird nach Deletion der T-DNA und benachbarter Abschnitte eines Vektors der pPZP -Familie (Hajdukiewicz et al., Plant Molecular Biology 25, 989-994. 1994) andere, die Border enthaltenden PCR Fragmente, eine Multiklonierungsstelle und einen unikalen Ort für die Einklonierung der Selektionsmarker benachbart zur linken Border derart eingebaut, daß der bakterielle Resistenzmarker des Vektors benachbart zur rechten Border ist. Dabei wird der erfindungsgemäße Vektor pSUN1 im Vergleich zum z. B. pPZP200 um 0,7 kb verkleinert.
Im zweiten Schritt werden mit 2 verschiedenen Deletionen alle Notl Orte und weitere z.T. nicht funktionelle Bereiche des Vektors entfernt (pSUN10). Dazu gehört auch der nic Erkennungsort, dem Transferorigin für den konjugalen Plasmidtransfer, ohne den eine Übertragung auf andere Bakterien durch natürliche Konjugation nicht mehr möglich ist. Dieses stellt hinsichtlich sicherheitsrelevanter Bedenken einen großen Vorteil gegenüber anderen Binärplasmiden dar, von denen nur das Plasmid pGreen als ein vom pUC abgeleiteter Vektor, der keinen nic Erkennungsort enthält, nicht mehr konjugierbar ist (Hellens et al., Plant Molecular Biology 42, 819-832. 2000). Die Entfernung dieses Plasmidabschnittes führte in *E. coli* bei dem Plasmid pAT153 gegenüber dem Plasmid pBR322 zu einer Steigerung der Kopienzahl von 1,5 - 3 fach (Twigg et al., Nature 283, 216-218. 1980).
In diesen Vektor der sogenannten zweiten Generation wurde bei der Entfernung des Ncol Ortes die 'overdrive' Sequenz des Ti Plasmids pTiA6 eingefügt. Diese Sequenz wirkt positiv in cis auf das Ausschneiden der T-DNA und damit auf den Transfer der T-DNA (Toro et al., Natl. Acad. Sci. USA 85, 8558-8562. 1985).

Die Manipulationen in diesem zweiten Schritt führen zu einer weiteren Reduktion der Größe des erfindungsgemäßen Vektors pSUN10.

Im dritten Schritt werden die Erkennungsstellen für Rekombinasen wie beispielsweise die FRT-Erkennungsorte für die FLP Rekombinase (Senecoff et al., The Journal of Biological Chemistry 261(16), 7380-7386. 1986. ) eingeführt, um ein Ausschneiden der verwendeten Selektionsmarker zu ermöglichen (erfindungsgemäße Vektor pSUN20).

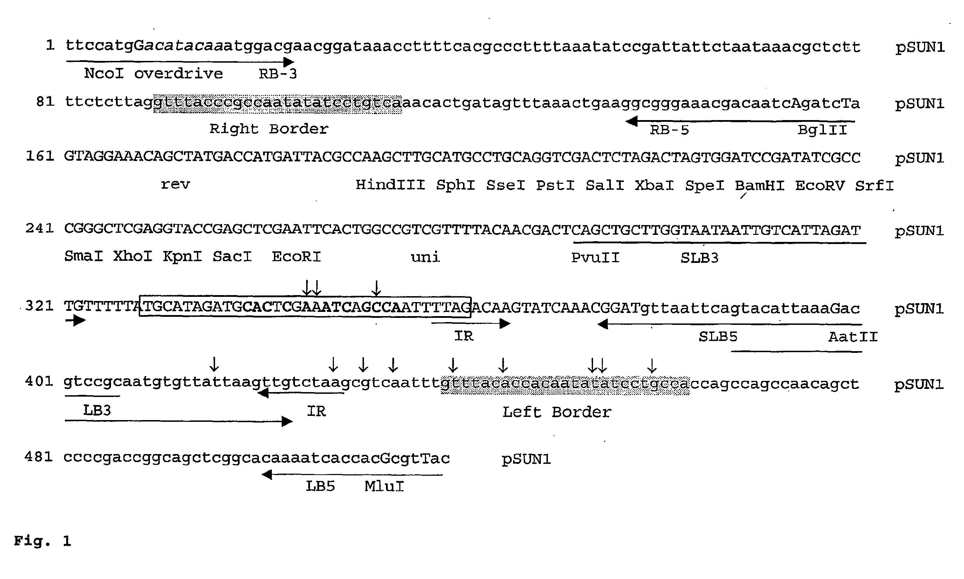

Gegenstand der vorliegenden Erfindung sind somit kleine Vektoren, vorzugsweise in Größen von 2 bis 12, besonders bevorzugt von 3-9, ganz besonders bevorzugt von 4-6 kb, zur effizienten Transformation von pflanzlichen Systemen enthaltend eine T-DNA, in die ggf. eine heterologe Nukleotidsequenz in einen umfangreichen Polylinker, der von üblichen Sequenzprimern umgeben ist, insertiert ist und welche von einer rechten und linken Border flankiert ist.
Ferner zeichnen sich die erfindungsgemäßen Vektoren dadurch aus, daß sie in der T-DNA Expressionskassetten zur Überexpression und/oder Repression von Fremdgenen enthalten können.
Die erfindungsgemäßen Binärvektoren zeichnen sich durch eine zusätzliche Sequenz (aBS, additional Border Sequenz) gemäß SEQ ID NO 1 aus. Diese aBS-Sequenz liegt benachbart zur linken Border in der T-DNA und ermöglicht eine saubere Integration der T-DNA in das Pflanzengenom.
Zur Verdeutlichung ist ein Ausschnitt von ca. 520 Nukleotiden des erfindungsgemäßen Vektors in Fig. 1 dargestellt. Die zusätzliche Sequenz aBS umfaßt erfindungsgemäß einen Bereich von ca. 60 bis 240 Nukleotiden, bevorzugt von 90 bis 120 Nukleotiden und besonders bevorzugt von ca. 120 Nukleotiden. Eine besondere Ausführungsvariante dieser erfindungsgemäßen zusätzlichen Border-Sequenz ist als SEQ ID No. 1 dargestellt. Ferner enthält die erfindungsgemäß zusätzliche Border-Sequenz ein funktionelles Sequenzmotif, welches in Fig. 1 durch einen Rahmen markiert ist. Innerhalb dieses Sequenzmotifs finden in verstärktem Maße Rekombinationsereignisse zwischen der T-DNA und dem Pflanzengenom statt. In einer besonders bevorzugten Ausführungsvariante der vorliegenden Erfindung umfaßt dieses Sequenzmotif einen Bereich von 33 Nukleotiden, der als SEQ ID NO. 2 dargestellt ist. In den verbleibenden Bereichen kann die erfindungsgemäß zusätzliche Nukleotidsequenz (aBS) verstärkt variieren, wobei sich ein erhöhter AT-Gehalt günstig auswirkt.
Unter funktionellen Äquivalenten sind erfindungsgemäß im wesentlichen gleichwirkende Nukleotidsequenzen zu verstehen. Funktionell äquivalente Sequenzen sind solche Sequenzen, welche trotz abweichender Nukleotidsequenz noch die gewünschten Funktionen besitzen. Funktionelle Äquivalente umfassen somit natürlich vorkommende Varianten der hierin beschriebenen Sequenzen sowie künstliche, z. B. durch chemische Synthese erhaltene Nukleotid-Sequenzen. Unter einem funktionellen Äquivalent versteht man insbesondere auch natürliche oder künstliche Mutationen einer ursprünglich isolierten Sequenz, welche weiterhin die gewünschte Funktion zeigen. Mutationen umfassen Substitutionen, Additionen, Deletionen, Vertauschungen oder Insertionen eines oder mehrerer Nukleotidreste.

Ferner werden beispielsweise auch solche Nukleotidsequenzen durch die vorliegende Erfindung mit umfaßt, welche man durch Modifikation der Nukleotidsequenz, resultierend in entsprechenden Derivaten, erhält. Ziel einer solchen Modifikation kann z. B. die weitere Eingrenzung der Sequenz oder z. B. auch die Einfügung weiterer Restriktionsenzym-Schnittstellen sein.
Weiterer Gegenstand der Erfindung sind somit Binärvektor gekennzeichnet durch Polylinker mit mehr als 6, vorzugsweise 15-20, besonders bevorzugt 16-25 unikalen Restriktionsschnittstellen.

Unter homologen Sequenzen sind erfindungsgemäß solche zu verstehen, die zu den erfindungsgemäßen Nukleotidsequenzen komplementär sind und mit diesen hybridisieren. Der Begriff hybridisierende Sequenzen schließt erfindungsgemäß substanziell ähnliche Nukleotidsequenzen aus der Gruppe von DNA oder RNA ein, die unter an sich bekannten stringenten Bedingungen eine spezifische Wechselwirkung (Bindung) mit den zuvor genannten Nukleotidsequenzen eingehen. Hierzu zählen auch kurze Nukleotidsequenzen mit einer Länge von beispielsweise 10 bis 30, bevorzugt 12 bis 15 Nukleotiden. Dies umfaßt erfindungsgemäß u. a. auch sogenannte Primer oder Sonden.
Ferner enthält der erfindungsgemäße Binärvektor eine T-DNA, begrenzt von den rechten und linken Borders, wobei beispielsweise der linken Border die erfindungsgemäß zusätzliche Sequenz benachbart sein kann. Die rechte Border kann ferner ein sogenanntes verstärkendes Segment oder "Overdrive-Sequenz" (Peralta et al., The EMBO Journal 5, 1137-1142. 1986) enthalten, welches sich günstig auf die Effizienz des T-DNA- Transfers und damit der darin enthaltenen gewünschten heterologen DNA auswirkt. In der beispielhaften Ausführung der in Fig. 1 dargestellten T-DNA befinden sich ferner beidseitig neben einem von den Uni- und Revers Primern flankierten umfangreichen Polylinker zusätzlich zwei singuläre Erkennungsstellen für Restriktionsenzyme. Diese können beispielsweise zur Integration von heterologen Nukleotidsequenzen, Selektionsmarkern und weiteren Modulen, wie z. B. Elementen für die Rekombination, alternativen Selektionssystemen (GST-MAT-System, (Sugita et al., The Plant Journal 22(5), 461-469. 2000) oder einem zweiten Selektionsmarker, zwischen Polylinker und Border genutzt werden. Zwei andere singuläre Restriktionsschnittstellen liegen in den erfindungsgemäßen Vektoren und Derivaten davon benachbart zur T-DNA und erlauben die Insertion von Markern oder Suizidgenen, welche die Überprüfung der Integration in die pflanzliche DNA erlauben. Diese Orte können auch für die Integration einer weiteren T-DNA genutzt werden und erlauben so die unabhängige Integration beispielsweise von Selktionsmarkern und Transgenen. Eine weitere Möglichkeit besteht in der Integration von *vir* Genen, die eine verbesserte Transformationseffizienz ermöglichen sollen (Ke et al., Plant Cell Reports 20, 150-156. 2001). Ferner zeichnet sich der erfindungsgemäße Vektor dadurch aus, daß er in der T-DNA Expressionskassetten zur Überexpression und/oder Repression von Fremdgenen enthalten kann. Ein weiterer Vorteil dieser Vektoren ist, daß das bakterielle Resistenzgen sich benachbart zur rechten Border und somit nach den mechanistischen Vorstellungen über den T-DNA Transfer von der übertragenen T-DNA weit entfernt befindet. Die erfindungsgemäßen Vektor pSUN10 und pSUN20 und ihre Derivate zeichnen sich erstens dadurch aus, daß sie durch Deletionen nicht benötigter Sequenzabschnitte im Vektorteil ohne die T-DNA nur eine Größe von 4,6 kb besitzen. Sie verfügen über alle Elemente, die für die Vermehrung in E.coli (ColE1 origin) und in den Agrobakterien (pVS1 origin) und für die Transformation (modifizierte linke und rechte Border) in die Pflanze notwendig sind. Die kleine Größe der erfindungsgemäßen Vektoren, das Fehlen häufig verwendeter Restriktionsorte außerhalb der T-DNA, der komfortable Polylinker, der ColE1 Origin und die damit verbundene hohe Kopienzahl der Plasmide ermöglichen eine sehr gute Klonierbarkeit anderer Sequenzabschnitte in die Vektoren. Die beiden flankierend zu der Multiklonierungsstelle liegenden weiteren unikalen Restriktionsorte Pvull und Bglll in der T-DNA sowie die beiden die T-DNA flankierenden Restriktionsorte Mlul bzw. Ncol (im pSUN10 Sspl) erhöhen die Modularität der Vektoren.
Die erfindungsgemäßen Binärvektoren sind gekennzeichnet durch die Kombination von geringer Größe des Vektors, das Vorhandensein aller für die Funktion notwendiger Elemente, das Fehlen von Erkennungsorten für gewöhnlich verwendete Restriktionsenzyme wie z.B. Notl außerhalb der T-DNA, die gute Handhabung durch die hohe Kopienzahl in E.coli und die Stabilität in den Agrobakterien, der sehr umfangreiche Polylinker mit 18 unikalen Restriktionsschnittstellen, das Vorhandensein von Erkennungsstellen für kommerziell erhältliche Sequenzierprimer flankierend zum Polylinker, die vom Polylinker unabhängige Klonierung des Selektionsmarkers neben die linke Border und die Lage des bakteriellen Selektionsmarkers benachbart zu rechten Border. Das fehlen der Notl Orte im Vektor eröffnet die Möglichkeit in der T-DNA eine spezielle Kassette zu etablieren, die das Klonieren von cDNA und genomischen Banken über die häufig genutzten EcoRI/Notl Adapter erlauben. Die erfindungsgemäße Kombination der Sequenzabschnitte und die damit verbundene hohe Modularität sind neu.
Des weiteren zeichnen sich die erfindungsgemäßen Binärvektoren dadurch aus, daß der Vektor pSUN20 und seine Derivate die Erkennungsmotive für die Rekombination FRT der FLP Rekombinase von dem 2µ Plasmid der Hefe Saccharomyces cerevisiae (Senecoff et al., The Journal of Biological Chemistry 261(16), 7380-7386. 1986) flankierend zur Insertionsstelle für die Selektionsmarker besitzt und so die Möglichkeit besteht, die Selektionsmarker aus den transgenen Linien durch Rekombination zu entfernen. Desweiteren besitzen sie eine unikale Restriktionsschnittstelle wie z.B. Scal zwischen dem Polylinker und der linken Border für die Insertion von Selektionsmarkern, die dann nach dem Zielgen in die Pflanze übertragen werden und so die Selektion von transgenen Pflanzen erlauben, die das Zielgen auch enthalten.

Aufgrund ihrer geringen Größe können die erfindungsgemäßen Binärvektoren auch effizient für den direkten Gentransfer eingesetzt werden. Hierfür kommen dem Fachmann bekannte Methoden in Betracht, wie z. B. Microinjektion, Elektroporation, Fusion von Protoplasten mit Liposomen, Behandlung von Protoplasten mit Calciumphosphat, Polyethylenglykol oder anderen Substanzen und Methoden, die ein Einschleusen der Binärvektoren in Pflanzenzellen fördern. Solche Verfahren werden z. B. in Römpp: Biotechnologie und Gentechnik, Thieme Verlag Stuttgart, 2. Aufl., 1999 auf den Seiten 324-330 beschrieben. In einer bevorzugten Variante wird beispielsweise die biolistische Methode eingesetzt, z. B. zur Überprüfung von Promotor-Reportergenfusionen mittels transienter Expression nach Beschuß mit DNA beladenen Goldpartikeln.
Der erfindungsgemäße Vektor bietet ferner aufgrund seines modularen Aufbaus zahlreiche Möglichkeiten zur Integration verschiedener Elemente, wie beispielsweise das codA-Gen aus *E*. *coli,* welches für eine Cytosindeaminase kodiert und eine kombinierte Positiv-Negativselektion erlaubt (Gallego et al., Plant Molecular Biology 39, 83-93. 1999), Sequenzen, welche die homologe Rekombination unterstützen, z. B. das cre/lox-System ( Sauer et al., Current Opinion in Biotechnology 5, 521-527. 1994.) oder das FLP Recombinase System (Maniatis, T., Fritsch, E. F. & Sambrook, J. Molecular cloning-A laboratory manual (Cold Springer Harbor Lab., Cold Springer Harbor, New York). 1982). Die singularen Restriktionsorte außerhalb der T-DNA im Vektor erlauben die Integration beispielsweise eines als Suizidgen wirkenden RNAse-Gens (Barnasegen; (Hanson et al., The Plant Journal 19(6), 727-734. 1999.) um die Übertragung von Vektorsequenzen zu unterdrücken. Außerdem bieten sie die Möglichkeit vir Gene zu integrieren, die eine erhöhte Transformationseffizienz in monocotylen Pflanzen ermöglichen sollen (Ke et al., Plant Cell Reports 20, 150-156. 2001). Weiterhin bieten sie beispielsweise auch die Möglichkeit eine zweite T-DNA zu integrieren, um nach Kotransformation markerfreie Pflanzen herzustellen.

Gegenstand der vorliegenden Erfindung ist somit auch ein Verfahren zur effizienten Transformation pflanzlicher mono- und dicotyler Systeme, wobei besonders kleine Vektoren, enthaltend eine T-DNA mit einem Polylinker mit sehr vielen, beispielsweise mit mehr als 6, vorzugsweise 15-20, besonders bevorzugt 16-25 unikalen unikalen Restriktionsschnittstellen, in die ggf. eine heterologe Nukleotidsequenz (gewünschtes Insert) inseriert ist und welche von einer rechten und einer dem pSUN1 entsprechenden modifizierten linken Border gemäß SEQ ID NO1 flankiert ist, in ein pflanzliches System übertragen wird und dieser Vektor mit hoher Effizienz eine Übertragung der von den rechten und linken Bordern flankierten T-DNA, in die ggf. die gewünschte heterologe Nukleotidsequenz inseriert ist, in das Genom eines pflanzlichen System vermittelt. Transformation von Pflanzen ist Routine für eine immer steigende Zahl von pflanzlichen Spezies, sowohl mono- als auch dicotylidoner Arten. Diese Erfindung betrifft auch solche pflanzlichen Spezies, die heute noch nicht zugänglich für genetische Transformationen sind. Im Prinzip kann diese Erfindung für jede Transformationsmethode, ob nun direkter oder indirekter Gentransfer, in entsprechende pflanzliche Zellen verwendet werden. Eine entsprechend der Erfindung bevorzugte Methode stellt der Agrobakterium vermittelte Gentransfer dar. Besonders bevorzugt ist die Verwendung der sogenannten Binärvektor Technologie die von den Patenten EP A120516 bzw. US 4.940.838 geschützt ist.

Die in diesem Verfahren verwendeten erfindungsgemäßen Vektoren enthalten als weitere wertvolle Elemente in der T-DNA Erkennungsstellen für gewöhnliche Sequenzprimer (pUC18 uni und revers Primer) sowie einen weiteren unikalen z. B. Sca I Ort für die Integration der Selektionsmarker benachbart zu linken Border, der beispielsweise von den FRT Erkennungsstellen der FLP Rekombinase des 2µ Plasmids von der Hefe Saccharomyces cerevisiae flankiert ist und ein späteres Entfernen der Selektionsmarker durch Rekombination ermöglicht. Der in diesem Verfahren verwendete Vektor pSUN20 und seine Derivate haben ebenfalls den Vorteil, daß in den Vektorsequenzen außerhalb der T-DNA keine üblichen Restriktionsschnittstellen wie z. B. Notl vorhanden sind. So könnten in die T-DNA Kassetten etabliert werden, die die Klonierung von Notl/EcoRl flankierten cDNA und genomischen Banken erlauben.Diese werden im folgenden als Genbibliotheken bezeichnet.
Unmittelbar vektorseitig der linken Border befindet sich ein Mlul Ort für die Integration von Negativmarkern, wie das codA-Gen aus *E*. *coli,* welches für eine Cytosindeaminase kodiert und eine kombinierte Positiv-Negativselektion erlaubt (Gallego et al., Plant Molecular Biology 39, 83-93. 1999).

Die vorliegende Erfindung umfaßt ferner ein transformiertes pflanzliches System, regenerierte Zellen oder Pflanze daraus, deren Nachkommen oder Samen davon hergestellt gemäß einem zuvor beschriebenen erfindungsgemäßen Verfahren. In einer besonderen Ausführungsvariante der vorliegenden Erfindung zeichnet sich dieses transformierte pflanzliche System dadurch aus, daß es keine Sequenzanteile des zuvor genannten Vektors außerhalb der rechten und/oder linken Border enthalten soll.

Gegenstand der vorliegenden Erfindung ist ferner die Verwendung des erfindungsgemäßen Binärvektors zur Erstellung von Genbibliotheken und deren Transformation von Pflanzen, bevorzugt vermittelt durch Agrobakterien.

Gegenstand der vorliegenden Erfindung ist auch eine Variante des Binärvektors , in welcher der Polylinker durch eine Abfolge seltener Restriktionsschnittstellen ersetzt wird und so insbesondere für das Einklonieren mehrerer Expressionskassetten (gene stacking) geeignet ist.

Die vorliegende Erfindung wird durch die nachfolgenden Beispiele näher charakterisiert, die jedoch nicht limitierend für die Erfindung sind:

### Allgemeine Methoden:

### 1. Klonierungsverfahren

Rekombinante DNA Techniken und Sequenzanalysen werden entsprechend Maniatis et al. ( Maniatis, T., Fritsch, E. F. & Sambrook, J. Molecular cloning-A laboratory manual (Cold Springer Harbor Lab., Cold Springer Harbor, New York). 1982) durchgeführt und die eingesetzten Enzyme nach Vorschrift angewendet. Als Basisvektor dient das Plasmid pPZP200 ( Hajdukiewicz et al., Plant Molecular Biology 25, 989-994. 1994). Die rechten und linken Borders wurden aus dem Plasmid pPZP200 bzw. aus dem Agrobakterienstamm C58(pTiC58) (DZMS 5172) amplifiziert.

### 2. Bakterienstämme

Für die Transformation in *E. coli* wurde der Stamm DH5α verwendet. Für die Transformation in Agrobakterien mittels der Frier-Tau-Methode wurden die Stämme EHA101, EHA 105, C58C1[mp90] LBA4404 und GV3101 verwendet (Höfgen et al., Nucl. Acids Res. 16(20), 9877. 1988).

### 3. Pflanzentransformation

Der Agrobakterien vermittelte Gentransfer von *Nicotiana tabacum* erfolgte durch die sogenannte "Blattscheibchenmethode" und von *Brassica napus* durch die Petiolentransformation (Moloney et al., Plant Cell Reports 8, 238-242. 1989).

### 4. Analyse der genomischen DNA aus transgenen Pflanzen

Die genomische DNA der transgenen Tabak und Raps Pflanzen wurde folgendermaßen präpariert:
Ca. 3-5 g Blattmaterial werden unter flüssigen Stickstoff zu einem feinen Puder gemörsert. Nach Überführen in ein 50ml Zentrifugenröhrchen und Zugabe von 15 ml Extraktionspuffer (500mM Natriumchlorid, 100mM Tris-HCl pH 8,0; 50mM EDTA , pH 8,0; 1mM Mercaptoethanol) wird der Extrakt gut vermischt. Dann wird 1 ml 20% SDS Lösung zu gegeben, geschüttelt und 10min bei 65°C inkubiert. Nach der Zugabe von 5ml 5M Kaliumacetat wird der Extrakt gemischt und 20 bis 30 min auf Eis gestellt. Nach der Zentrifugation 20 min bei 12000 rpm wird der Überstand durch eine Miracloth Membran in ein neues Zentrifugenglas überführt. Nach der Zugabe von 10ml Isopropanol wird gemischt und 20 bis 30 min bei -20°C gefällt. Die genomische DNA wird 20 min bei 10000 rpm abzentrifugiert und der Überstand verworfen. Das getrocknete Pellet wird in 0,7 ml 50xTE resuspendiert und in ein Tube überführt. Dann wird die RNA nach Zugabe von 20µl RNase (10mg/ml), Inkubation 30 min bei 37°C, Zugabe von 75µl 3M Natriumacetat, Mischen und Zentrifugation für 15min bei 13000rpm entfernt. Der Überstand wird in ein neues Tube transferiert und 500µl Isopropanol dazugeben. Nach Fällung bei Raumtemperatur von 5min wird die genomische DNA 15min bei 10000rpm abzentrifugiert und mit 70% Ethanol gewaschen. Das getrocknete Pellet wird in 200µl TE bei 4°C über Nacht gelöst, die Konzentration und die Qualität der genomischen DNA bestimmt.
Alternativ wurde genomische DNA mit dem DNeasy Plant Kit von Quiagen isoliert.
In einem ersten Schritt wurden die transgenen Linien über PCR mit genspezifischen Primern identifiziert. Die Integration der Fremd-DNA wurde mittels "Southernblot" - Analysen von 20µg DNA nach geeigneter Restriktionsspaltung untersucht. Mittels dem "Universal Genome Walker Kit" von Clontech wurden Übergänge zwischen der T-DNA und pflanzlicher DNA isoliert und anschließend sequenziert.

### 5. β-Glucuronidase - Aktivitätstest (GUS - Assay)

Das Reportergen β-Glucuronidase ist ein bakterielles Enzym, das sowohl quantitativen als auch histochemischen Aktivitätsbestimmungen zugänglich ist. Gewebeproben wurden in 1mM X-Gluc, 50mM Na-Phosphat (pH 7,0) und 0,1% Tween 20 über Nacht bei 37°C inkubiert und dann ausgewertet. Die Glucuronidaseaktivität in den transgenen Linien wurden wie beschrieben (Jefferson, et al., Plant Molec. Biol. Rep. 5, 387-405. 1987) nach Extraktion der Gewebe durch den Umsatz von 4-Methylumbelliferyl-β-D-glucuronid quantitativ bestimmt.

### 6. Luciferase Assay:

Die Expression des Luciferasegens wurde mit dem Luciferase Assay System (E1500) von Promega entsprechend der Vorschrift analysiert.

### Konstruktion des erfindungsgemäßen Binärvektors pSUN1

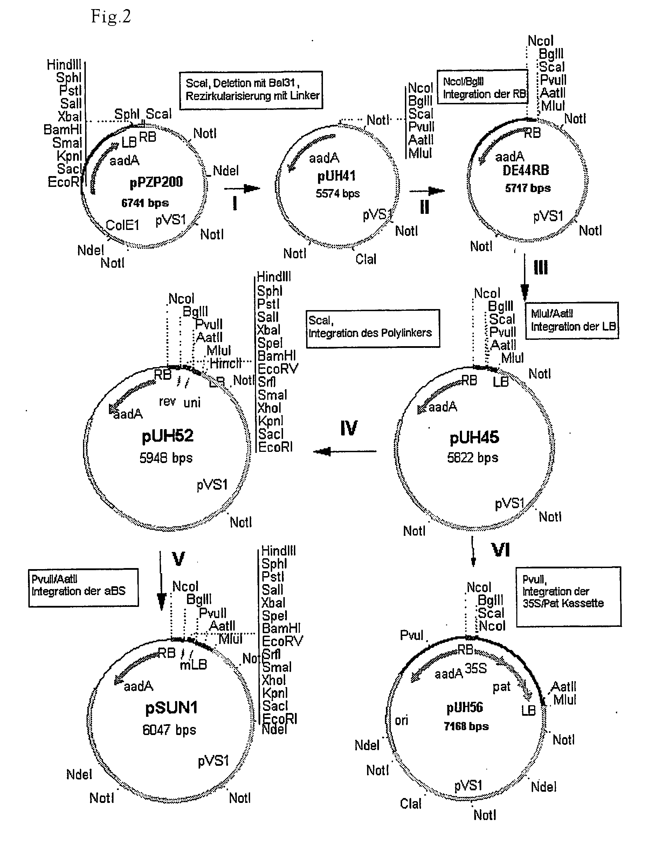

Nach Linearisierung des Plasmids pPZP200 (Hajdukiewicz et al., Plant Molecular Biology 25, 989-994. 1994) mit Scal wurden die T-DNA und benachbarte Sequenzabschnitte mit Nuklease Bal31 abgebaut. Nach Glättung der Enden mit dem Klenow Fragment der DNA Polymerase wurde das verbliebene Plasmid unter Verwendung des Linkers 5'-ttccatggtcagatctagtactcagctgagacgtcttacgcgtt-3' rezirkularisiert (Schritt I). Bei der Rezirkularisierung wurde das Plasmid pUH41 gebildet. (Fig. 2) Auf diesem Linker befinden sich unikale Restriktionsorte, in die später die Border, der Polylinker und Selektionsmarker eingebaut werden.
Bei der anschließenden Transformation von *E. coli* und Selektion auf Spectinomycin (100mg/l) wuchsen nur Klone, die das vollständige Resistenzgen aadA Aminoglycoside-3"-adenylransferase enthalten.

Die Analyse dieser Kolonien ergab, das der Klon pUH41 die umfangreichste Deletion besaß, eine sehr gute Resistenz aufwies und sich gut in den Agrobakterienstamm EHA105 transformieren ließ. In diesen Klon wurden die Border eingefügt.
Dazu wurden die rechte und linke Border mittels Advantage Tth Polymerase (Clontech) und den Primern RB5/RB3 bzw. LB5/LB3 aus dem Plasmid pPZP200 (Hajdukiewicz et al., Plant Molecular Biology 25, 989-994. 1994) amplifiziert. Nach Klonierung der PCR Fragmente in den Vektor pUC18 wurde die Sequenzen verifiziert. Die zur Amplifizierung der rechten und linken Border eingesetzten Primer sind nachfolgend aufgeführt:
Primer RB5: 5'- gagcttagatctgattgtcgtttcccgccttc-3';
Primer RB3: 5'-cctgtggttgccatggacatacaaatggacg-3',
Annealing-Temperatur (Ta) = 56°C

Primer LB5: 5'- ctgatgggctgcctgtaacgcgtggtgattttg-3';
Primer LB3: 5'-cattaaagacgtccgcaatgtgttattaagttg-3',
Annealing Temperatur Ta = 50°C

Die rechte Border wurde über NcoI/BgIII in den Polylinker des Vektors pUH41 kloniert (Fig.2, Schritt II). In das resultierende Plasmid pDE44RB wurde nach einer Aatll/Mlul Spaltung die linke Border eingefügt (Schritt III). In den Pvull Ort des entstandenen Plasmid pUH45 wurde die 35S Promotor-Phosphinothricinacetyltransferase-35SpA Kassette eingebaut, um in einer anschließenden Tabaktransformation die Integration und die Regeneration zu überprüfen (pUH56, Fig.2, Schritt VI).

Um zahlreiche unikale Restriktionsorte im Polylinker nutzen zu können, wurde der Linker 5'-gtacctcggcccgggcgatatcggatccactagt-3' in den mit XbaI und Asp718 geschnittenen Vektor pUC19 einkloniert. Der Polylinker, der die Orte EcoRI SacI KpnI XhoI SrfI SmaI EcoRV BamHI Spel XbaI SaII HincII PstI SseI SphI HindIII enthält, wurde über die universal und reverse M13 Primer amplifiziert und in den ScaI Ort des pUH45 ligiert (Schritt IV).

Entsprechend der beiden möglichen Orientierungen des Polylinker entstanden dabei die Plasmide pUH52 (Fig. 2) und pUH53 (nicht dargestellt).
Zur Isolation der zusätzlichen Sequenz (additional left border Sequenz) wurde eine PCR Amplifikation mittels folgender Primer
SLB5: 5'- gcggacgtctttaatgtactgaattaacatccg - 3'
SLB3: 5'- cacagctgcttggtaataattgtcattagattg - 3'
an der isolierten Plasmid DNA des DZMZ - pTi Plasmids des *Agrobacterium tumefaciens* Stammes C58 (DSM no.: 5172) bei einer Ta von 55°C durchgeführt. Das PCR Produkt wurde in das Plasmid pUC18Smal kloniert. Der Klon pUH46 hatte entsprechend der Sequenzanalyse die richtige Sequenz.
In die Plasmide UH52 und UH53 wurde nach einer Pvull/Aatll Spaltung das Pvull/Aatll Fragment des Plasmids UH46 und damit die erfindungsgemäße additional border Sequenz eingebaut, resultierend in den Vektoren pSUN1 (Fig. 2, Schritt V) und pUH58 (nicht dargestellt).

### Transformation des Plasmids pUH56 in Tabak

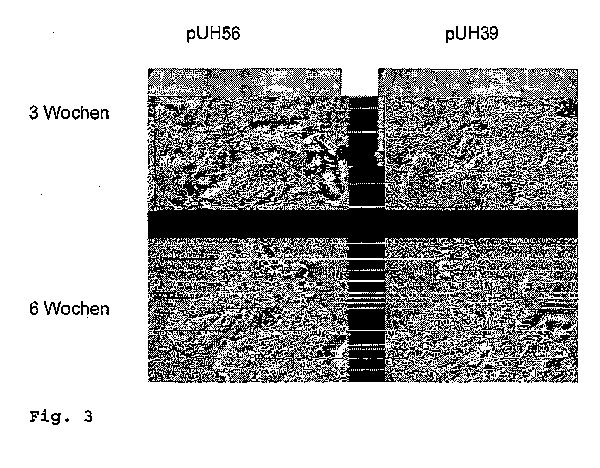

Um zu testen, ob die Veränderung der Border einen Einfluß auf die Kallusbildung und Regeneration hat, wurden die Plasmide pUH56 (Fig.2), welches eine 35S-Phosphinotricinacetyltransferase Kassette im Plasmid UH45 (Fig.2) hat, und zur Kontrolle pPZP200::35SPat (entspricht dem Vektor pUH 39) in den Agrobakterienstamm EHA 105 transformiert. Die Transformation von *Nicotiana tabacum* erfolgte mit der Blattscheibchenmethode. In der Fig. 3 sind die regenerierenden Sprosse 3 und 6 Wochen nach der Transformation im Vergleich zur Kontrolle (pUH39) zu sehen. Die Regeneration verlief ohne feststellbare Unterschiede zwischen den Regeneraten. Die Veränderung der Border hatte somit keinen negativen Einfluß auf die Regeneration.

### Überprüfung der Transformationseffizienz und der korrekten Insertion der T-DNA

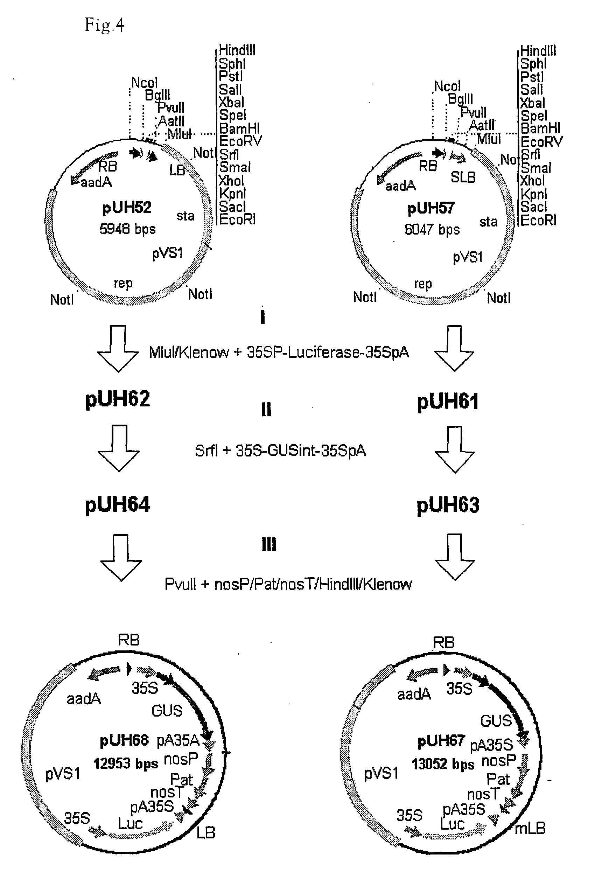

Die Expression der Reportergene Glucuronidase und Luciferase wurde genutzt, um zum einen die Effizienz der neuen Vektoren bei der Übertragung eines Transgens (Glucuronidase, GUS) zu ermitteln und zum anderen durch den Einbau vektorseitig zur linken Border der T-DNA (Luciferase, Luc) nachzuweisen, daß keine Vektorsequenzen mit übertragen worden sind. Die zum Einbau der Kassetten 35S Promotor/Glucuronidase und 35S Promotor/Luciferase notwendigen Klonierungsschritte sind in der Fig.4 nachzuvollziehen.
pUH52 und pSUN1 wurden mit MIuI linearisiert, geglättet, dephosphoryliert und mit dem geglätteten HindIII Fragment aus dem Vektor pRT101Luc (Maas et al., Plant Mol. Biol. 16, 199-207. 1991) ligiert (Schritt I). In den SrfI Ort der resultierenden Plasmide pUH62 und entsprechend pUH61 wurde dann das geglättete EcoRI/HindIII Fragment des Plasmids pGUSINT37 kloniert, die Plasmide pUH64 und entsprechend pUH63 erzeugend (Schritt II). In diese Konstrukte wurden in den Pvull Ort als Selektionsmarker die nosP/Pat/nosT (HindIII, geglättet) bzw. die nosP/NPTII/nosT (Hindlll/BamHl, geglättet) Kassetten integriert (Schritt III). Die daraus resultierenden Klone pUH68 und pUH67 bzw. pUH76 und pUH77 wurden in die Agrobakterienstämme EHA101 bzw. GV3101 und dann in Tabak, *Arabidosis* bzw. *Brassica napus* transformiert.
Die transformierten Pflanzen wurden im Gewächshaus angezogen, wobei 70 - 90% der Sprosse Wurzeln bildeten. Die selektierten Pflanzen waren zu 80-100% transgen für den Selektionsmarker. Die Glucuronidase- und Luciferaseaktivitäten wurden wie oben bestimmt. 70-90% der Pflanzen zeigten GUS Aktivität und genomische PCR erwies die Integration des GUS Gens in 90-100% der analysierten Pflanzen. Damit ist die Korrelation zwischen den resistenten und die Glucuronidase exprimierenden Pflanzen sehr hoch, was bedeutet, daß die T-DNA vollständig übertragen worden ist. Dies wurde erreicht durch die Klonierung der Selektionsmarker benachbart zur linken Border. Da der Transfer der T-DNA an der rechten Border beginnt, wird zuerst das Reportergen und dann das Gen des Selektionsmarkers übertragen (Becker et al.,Plant Mol. Biol. 20, 1195-1197. 1992). Dies wird auch bestätigt durch genomische PCR- und Southern - Analysen.
Eine eventuelle Luciferaseaktivität in den transgenen Pflanzen bedeutet, daß zumindest dieser Teil des Vektor in das Genom integriert ist. Bei der Analyse der mit den erfindungsgemäßen Vektoren pUH67 und pUH68 transformierten transgenen Tabak Pflanzen wurden keine Luciferaseaktivitäten detektiert. Allerdings zeigten ca. 30% der analysierten Rapspflanzen Luciferaseaktivität. Genomische Analysen bestätigten, daß 30-50% der transgenen Pflanzen auch Vektorsequenzen enthalten können. Dies stimmt mit Literaturdaten überein (Martineau et al., The Plant Cell 6, 1032-1033. 1994, Ramanathan et al., Plant Mol.Biol. 28, 1149-1154. 1995, Kononov et al., The Plant Journal 11, 945-957. 1997)

### Konstruktion des erfindungsgemäßen Binärvektors pSUN10

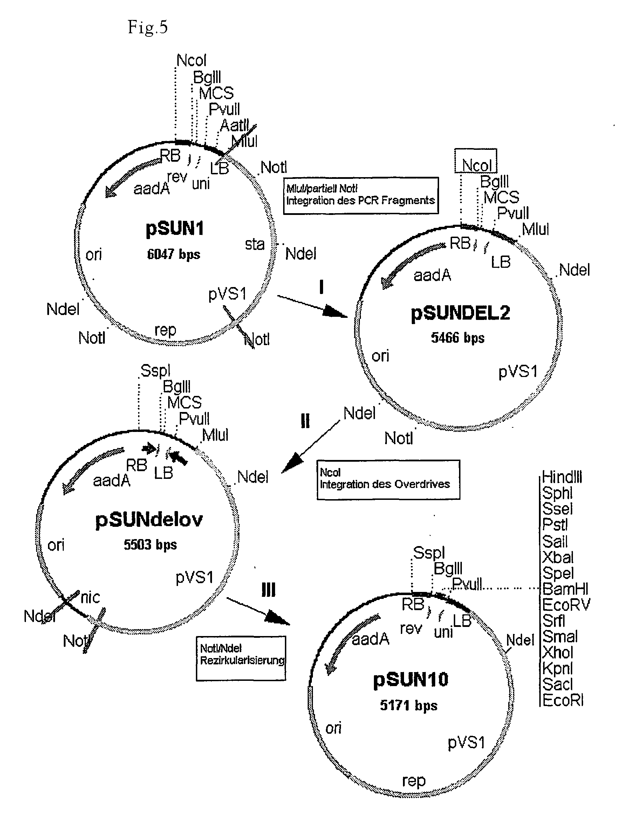

Im Klonierungsschritt I (Fig. 5) wurde über PCR ein Fragment des pVS1 Origin aus dem Plasmid pSUN1 amplifiziert. Die verwendeten Primer PVS5a 5'-cgagcgacgcgtctaaaaaggt-3' und PVSBsp 5'-caggggccccttgccacgattcaccgggg-3' entsprechen den Positionen 1093-1105 und 2390-2371 auf dem Plasmid pSUN1. Dieses mit Mlul/BSP120l gespaltene PCR Fragment wurde anstelle des deletierten 1878 bp Fragmentes in das Plasmid pSUN1, welches zuvor mit Mlul und partial mit Not I gespalten, kloniert. Dabei entstand das Plasmid pSundel. Die overdrive Sequence des Octopin Plasmids pTiA6 ( Toro et al., Proc. Natl. Acad. Sci. USA 85, 8558-8562. 1985) wurde mit Hilfe der Oligos ov_RB 5'-catgataagtcgcgctgtatgtgtttgtttgaatatt3' und ov_Ssp 5'-catgaatattcaaacaaacacatacagcgcgacttat-3' in den NcoI Ort kloniert, der dabei entfernt wurde (Schritt II). Als neuer unikaler Restriktionsort benachbart zur rechten Border in dem entstandenen Plasmid pSUNdelov steht der Sspl Ort zur Verfügung, um an diese Stelle weitere bakterielle Selektionmarker, T-DNA's oder andere Module einzusetzen.
Im Klonierungsschritt III wurde durch die Spaltung mit Notl und Partialspaltung mit Ndel, Glättung der Enden und Rezirkularisierung des Plasmids weitere 336 bp und der nic-Ort, der Origin für Konjugationen mit anderen Bakterien, deletiert. Somit können diese Vektoren und ihre Derivate nicht mehr über Konjugation verbreitet werden, welches die biologische Sicherheit wesentlich erhöht.
Dabei entstand das Plasmid pSUN10, welches sich vom Plasmid pSUN1 durch die Deletion zweier Sequenzabschnitte inklusiv des nic-Ortes und den integrierten overdrive des Ti-Plasmids pTiA6 unterscheidet. Die T-DNA wurde dabei nicht verändert.

### Konstruktion des erfindungsgemäßen Binärvektors pSUN20

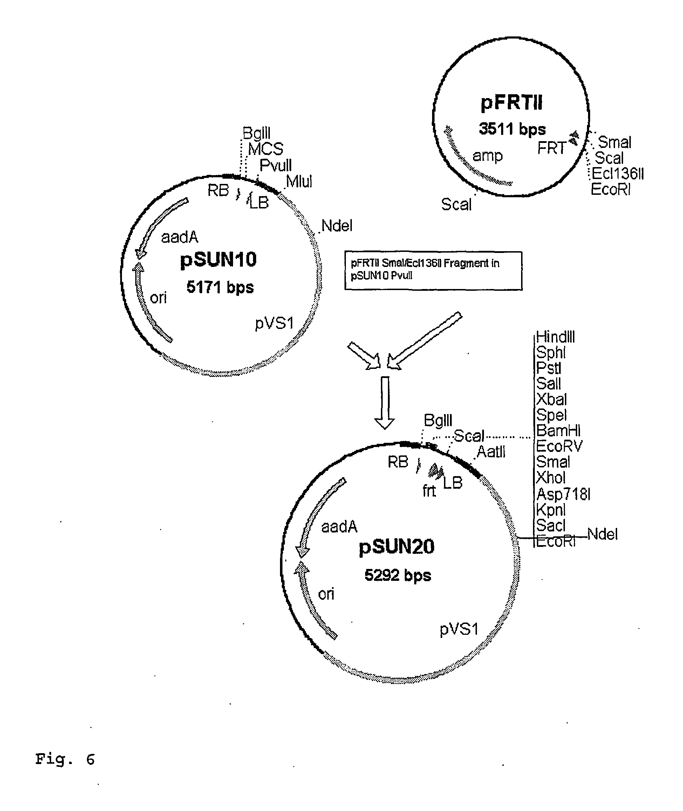

Ziel dieser Klonierung war es, die Möglichkeit zu besitzen, den Selektionsmarker aus den transgenen Linien wieder zu entfernen. Als Beispiel wurde hier das FLP/FRT Rekombinase System aus Hefe verwendet. (Senecoff et al., The Journal of Biological Chemistry 261 (16), 7380-7386. 1986). Dazu mußten die FRT Erkennungsstellen mit Hilfe von Oligos synthetisiert und kloniert werden. Dazu wurde zuerst eine Erkennungstelle mit Hilfe der Oligos 5'-gaagttcctatactttcttgagaataggaacttcggaataggaacttcgtcgacgtac-3', 5'-cagtcgacgaagttcctattccgaagttcctattctcaagaaagtataggaacttcgtac-3' erzeugt und in den Kpnl Ort des Plasmids pOCS1 kloniert. In den Sall Ort flankierend zur ersten FRT- Erkennungsstelle wurde dann die zweite Erkennungsstelle, ebenfalls aus Oligos erzeugt, über Xhol Enden kloniert. Die Oligos haben folgende Sequenzen: FRTII-1 5'-tcgagtactgaagttcctatactttcttgagaataggaacttcggaatag-3' und FRTII-2 5'-tcgagtgaagttcctattccgaagttcctattctcaagaaagtataggaa-3'. Dabei entstand das Plasmid pFRTII, dessen Sequenz verifiziert wurde. Die fusionierten Erkennungstellen wurden dann als Smal/Ecl136II Fragment ausgeschnitten und in den Pvull Ort des pSUN10 kloniert (Fig. 6).

Das dabei entstandene Plasmid pSUN20 unterscheidet sich somit vom pSUN10 nur durch die zusätzliche Sequenz der FRT Erkennungsorte von dem Plasmid pSUN10. Der zwischen den FRT Erkennungstellen befindliche ScaI Ort wird für die Klonierung des Selektionsmarker genutzt.

### Nachweis der stabilen Vermehrung in Agrobakterium tumefaciens

Da Teile des pVS1 Sequenzabschnittes deletiert wurden, soll hier gezeigt werden, daß dies keinen Einfluß auf die Vermehrung und die Stabilität des erfindungsgemäßen Binärplasmides in den Agrobakterien hat.
Der erfindungsgemäße Vektor pSUN10 wurde mittels Einfrieren/Auftauen in die Agrobakterienstämme Stämme EHA101, EHA 105, C58C1[mp90] LBA4404 und GV3101 transformiert. Nach ca. 2 Tagen waren die Kolonien normal gewachsen. Von den Kolonien des EHA 101/pSUN10 wurden 3 ausgewählt, in 5ml YEB Medium mit 50mg/l Kanamycin und 100mg/l angezogen und die Plasmid - DNA isoliert. Nach Spaltung und Verifizierung wurden aus diesen Kulturen jeweils 100µl genommen, um 100ml YEB-Medium an zu impfen. Pro Probe wurde jeweils ein Kolben mit Kanamycin/Spectinomycin und einer mit Kanamycin versehen. Im ersten Kolben konnten aufgrund der Spectinomycinresistenz nur Agrobakterien wachsen, die das Binärplasmid enthalten, im zweiten Kolben konnten auch EHA 101 Zellen wachsen, die das Plasmid verloren hatten.
Nach einer Übernachtkultur wurden die Agrobakterien auf Petrischalen mit YEB -Medium entsprechend ihren Resistenzen in verschiedenen Verdünnungen ausplattiert. Die Petrischalen mit den Verdünnungen von 10⁻⁸ und 10⁻⁹ wurden ausgezählt. Diese Prozedur wurde noch 5 mal wiederholt, so daß 6 Generationen analysiert wurden. Von der letzten Generation wurde von allen Proben Plasmid DNA isoliert, deren Qualität und Quantität sich nicht unterscheidet.

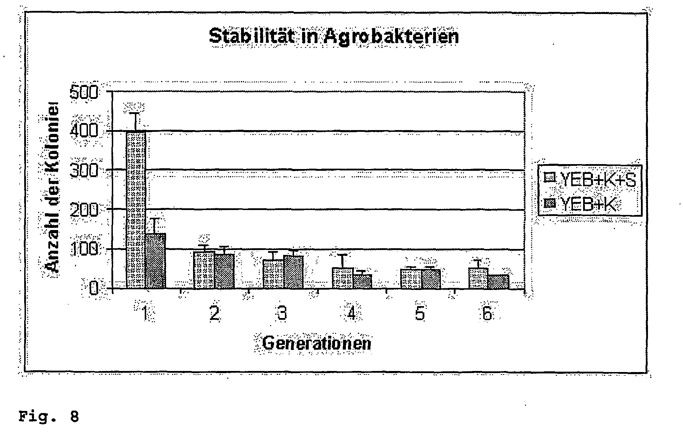

In der Fig. 8 sind in einem Diagramm die Daten zusammengefaßt. Die Höhe der Balken zeigt die ausgezählten Kolonien und es konnte kein signifikanter Unterschied zwischen den unter für das Binärplasmid pSUN10 nicht selektiven Bedingungen und unter selektiven Bedingungen festgestellt werden.

### Konstruktion des pSUN20Test

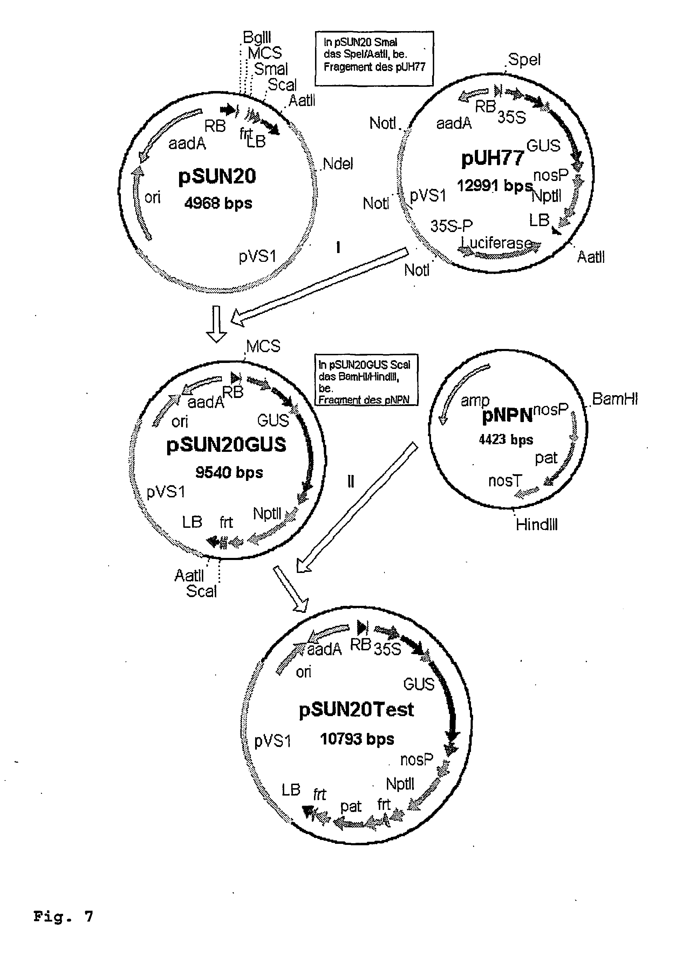

Entsprechend der Fig. 7 wurde ein Konstrukt für den Test des neuen erfindungsgemäßen Vektors pSUN20 hergestellt.
In den Smal Ort des Vektors pSUN20 wurde das Spel/Aatll Fragment des Plasmids UH77 welches ein intronhaltiges GUS Gen unter der Kontrolle des 35S Promotors und das NPTII Gen unter der Kontrolle des NosP enthält, kloniert (Schritt I). In den Scal Ort des resultierenden Plasmids pSUN20GUS zwischen die Erkennungsorte der FLP Rekombinase wurde der Selektionsmarker Phosphinothricinacetyltransferase unter der Kontrolle des nosP kloniert (Schritt II).
Das resultierende Plasmid wurde in Tabak transformiert.
Die transformierten Pflanzen wurden im Gewächshaus angezogen und die Glucuronidaseaktivität wie oben bestimmt. Die Korrelation zwischen den Phosphinotricin und NPTII resistenten und die Glucuronidase exprimierenden Pflanzen war sehr hoch, so daß die T-DNA vollständig übertragen worden ist. Dies wird auch bestätigt durch die genomischen PCR- und Southern - Analysen. Die Nachkommenschaften wurden mit Rekombinase exprimierenden Pflanzen gekreuzt und in den nicht mehr Phosphinothricin resistenten Pflanzen die GUS Expression analysiert. Es konnte gezeigt werden, daß diese Pflanzen das Zielgen enthalten und die Excision des Selektionmarker erfolgreich war.

### Figurenbeschreibung

Fig. 1: Sequenzausschnitt aus dem erfindungsgemäßen Vektor pSUN1 enthaltend die erfindungsgemäß zusätzliche Sequenz aBS
Hierbei haben die verwendeten Abbkürzungen folgende Bedeutung:
RB-3; RB-5: Primer für die Amplifikation der Rechten Border
LB-3; LB-5: Primer für die Amplifikation der Linken Border
aBS-3; aBS-5: Primer für die Amplifikation der erfindungsgemäß zusätzlichen Sequenz; additional Border Sequence (aBS)
Rev: reverse Sequenzprimer
Uni: universeller Sequenzprimer
IR: inverted repeat
↓: Übergangsstellen von T-DNA in das Genom transgener Pflanzen

Fig. 2: Schematische Darstellung der Konstruktion des Binärvektors pSUN1.
Hierbei haben die verwendeten Abbkürzungen folgende Bedeutung:
AadA: Spectinomycin/ Streptomycin-Resistenz
RB: Rechte Border
aBS: zusätzliche Nukleotidsequenz; additional Border Sequence
sta: partitioning protein
rep: pVS1 Replikationsprotein
pVS1: Replikationsstart mit einem breiten Wirtsspektrum des Plasmids pVS1
ori: Replikationsstart ColE1
Ferner sind singuläre Erkennungsstellen für Restriktionsenzyme eingezeichnet.
Fig. 3: Vergleich der Regeneration von mit den Vektoren pUH56 und pUH39 (Kontrolle) transformiertem Tabak nach 3 bzw. 6 Wochen.
Fig. 4: Schematische Darstellung der Konstruktion der Binärvektoren pUH68 und pUH67
Hierbei haben die verwendeten Abbkürzungen folgende Bedeutung:

| | |
|---|---|
| AadA: | Spectinomycin/ Streptomycin-Resistenz |
| RB: | Rechte Border |
| LB: | Linke Border |
| aBS: | erfindungsgemäß zusätzliche (additional) Border-Sequenz |
| 35S: | Promotor der 35S RNA des Blumenkohlmosaikvirus |
| pA35S: | Terminator der 35S RNA des Blumenkohlmosaikvirus |
| nosP: | Promotor des Nopalinsynthase-Gens aus Agrobacterium tumefaciens |
| nosT: | Transkriptionsterminator des Nopalinsynthase-Gens aus Agrobacterium tumefaciens |
| GUS: | Reportergen, kodierend für die β-Glucuronidase aus E. *coli* |
| Luc: | Reportergen, kodierend für die Luciferase aus Firefly |
| Pat: | Gen der Phosphinotricinacetyltransferase, synthetisch |
| rep: | pVS1 Replikationsprotein |
| pVS1: | Replikationsstart mit einem breiten Wirtsspektrum des Plasmids pVS1 |
| ori: | Replikationsstart ColE1 |

Ferner sind singuläre Erkennungsstellen für Restriktionsenzyme eingezeichnet.
Fig. 5 Schematische Darstellung der Konstruktion des Binärvektors pSUN10
Hierbei haben die verwendeten Abbkürzungen folgende Bedeutung:

| | |
|---|---|
| aadA: | Spectinomycin/ Streptomycin-Resistenz |
| RB: | Rechte Border |
| LB: | Linke Border |
| MCS: | Multiklonierungsstelle |
| pVS1: | Replikationsstart mit einem breiten Wirtsspektrum des Plasmids pVS1 |
| ori: | Replikationsstart ColE1 |

Ferner sind singuläre Erkennungsstellen für Restriktionsenzyme eingezeichnet.
Fig.6. Schematische Darstellung der Konstruktion des Binärvektors pSUN20
Hierbei haben die verwendeten Abbkürzungen folgende Bedeutung:

| | |
|---|---|
| aadA: | Spectinomycin/ Streptomycin-Resistenz |
| RB: | Rechte Border |
| LB: | Linke Border |
| MCS: | Multiklonierungsstelle |
| pVS1: | Replikationsstart mit einem breiten Wirtsspektrum des Plasmids pVS1 |
| ori: | Replikationsstart ColE1 |
| frt: | Erkennungsorte für die FLP Rekombinase aus Hefe |

Ferner sind singuläre Erkennungsstellen für Restriktionsenzyme eingezeichnet.
Fig. 7 Schematische Darstellung der Konstruktion des Binärvektors pSUN20Test

| | |
|---|---|
| AadA: | Spectinomycin/ Streptomycin-Resistenz |
| RB: | Rechte Border |
| LB: | Linke Border |
| aBS: | erfindungsgemäß zusätzliche (additional) Border-Sequenz |
| 35S: | Promotor der 35S RNA des Blumenkohlmosaikvirus |
| pA35S: | Terminator der 35S RNA des Blumenkohlmosaikvirus |
| nosP: | Promotor des Nopalinsynthase-Gens aus Agrobacterium tumefaciens |
| nosT: | Transkriptionsterminator des Nopalinsynthase-Gens aus Agrobacterium tumefaciens |
| GUS: | Reportergen, kodierend für die β-Glucuronidase aus *E*. *coli* |
| NptII | Gen der Neomycinphophostransferase, |
| Pat: | Gen der Phosphinotricinacetyltransferase, synthetisch |
| rep: | pVS1 Replikationsprotein |
| pVS1: | Replikationsstart mit einem breiten Wirtsspektrum des Plasmids pVS1 |
| ori: | Replikationsstart ColE1 |
| frt. | Erkennungsorte für die FLP Rekombinase aus Hefe |

Ferner sind singuläre Erkennungsstellen für Restriktionsenzyme eingezeichnet.
Fig.8 Darstellung der Stabilität der Agrobakterien

| | |
|---|---|
| YEB: | Medium |
| K: | selektiert auf Kanamycin |
| K+S: | selektiert auf Kanamycin und Spectinomycin |

## Patentansprüche

1. Binärvektor zur effizienten Transformation von pflanzlichen Systemen, enthaltend eine T-DNA, in die eine heterologe Nukleotidsequenz inseriert ist und welche von einer rechten und linken Border flankiert ist, wobei der Vektor benachbart zur linken Border die zusätzliche Sequenz gemäß SEQ ID NO 1, und einen Polylinker zwischen den Border mit mehr als 6 unikalen Restriktionsstellen aufweist.

2. Binärvektor gemäß Anspruch 1 **dadurch gekennzeichnet, dass** der Vektor eine Größe von 2 bis 12, bevorzugt von 3-9, besonders bevorzugt von 4-6 kb besitzt.

3. Binärvektor nach einem der Ansprüche 1-2 **gekennzeichnet durch** Polylinker mit 15-20, besonders bevorzugt 16-25 unikalen Restriktionsschnittstellen.

4. Binärvektor nach einem der Ansprüche 1-3 **gekennzeichnet durch** das Vorhandensein von Erkennungsstellen für Sequenzprimer pUC18 uni und revers Primer flankierend zum Polylinker.

5. Binärvektor nach einem der Ansprüche 1-4 **gekennzeichnet durch** die vom Polylinker unabhängige Klonierung des Selektionsmarkers neben die linke Border.

6. Binärvektor nach einem der Ansprüche 1-5 **gekennzeichnet durch** die Lage des bakteriellen Selektionsmarkers benachbart zur rechten Border.

7. Binärvektor nach einem der Ansprüche 1-6 **gekennzeichnet durch** erhöhte Biosicherheit durch Entfernung einer Region die den nic-Ort enthält.

8. Binärvektor gemäß Anspruch 1-7 **dadurch gekennzeichnet, dass** er die Erkennungsmotive für Rekombinasen flankierend zur Insertionsstelle für die Selektionsmarker besitzt.

9. Binärvektor zur effizienten Transformation von pflanzlichen Systemen, enthaltend eine T-DNA, in die eine heterologe Nukleotidsequenz inseriert ist und welche von einer rechten und linken Border flankiert ist, wobei der Vektor benachbart zur linken Border eine zusätzliche Sequenz enthaltend ein Sequenzmotif gemäß SEQ ID NO 2 oder ein Sequenzmotif mit Homologien von wenigstens 80 %, bevorzugt 90 % zu SEQ ID NO 2 und einen Polylinker zwischen den Border mit mehr als 6 unikalen Restriktionsstellen aufweist.

10. Verfahren zur Herstellung eines Binärvektors gemäß einem der Ansprüche 1-9 **dadurch gekennzeichnet, dass**
a. nach Deletion der T-DNA und benachbarter Abschnitte eines Vektors der pPZP-Familie andere, die Border enthaltenden PCR Fragmente, eine Multiklonierungsstelle und einen unikalen Ort für die Einklonierung der Selektionsmarker eingebaut werden;
b. durch 2 verschiedene Deletionen alle NotI Orte und weitere z.T. nicht funktionelle Bereiche des Vektors entfernt werden;

11. Verfahren nach Anspruch 10 **dadurch gekennzeichnet, dass** nach Schritt (b) die Erkennungsstellen für Rekombinasen eingeführt werden.

12. Verfahren nach den Ansprüchen 10 oder 11 **dadurch gekennzeichnet, dass** im ersten Schritt (a) der bakterielle Resistenzmarker benachbart zur rechten Border eingebaut wird.

13. Verwendung eines Binärvektors gemäß einem der Ansprüche 1-9 zur effizienten Transformation pflanzlicher Systeme.

14. Verwendung eines Binärvektors gemäß einem der Ansprüche 1-9 zur Herstellung von transformierten pflanzlichen Systemen, regenerierten Zellen oder Pflanzen daraus, deren Nachkommen oder Samen.

15. Verwendung eines Binärvektors gemäß einem der Ansprüche 1-9 zur Erstellung von Genbibliotheken.

16. Verwendung eines Binärvektors gemäß einem der Ansprüche 1-9 zur Transformation von Pflanzen, bevorzugt vermittelt durch Agrobakterien.

## Claims

1. A binary vector for the effective transformation of plant systems, comprising a T-DNA into which a heterologous nucleotide sequence is inserted and which is flanked by a right and a left border, where the rector comprises, adjacent to the left border, the additional sequence as shown in SEQ ID NO 1, and, between the borders, a polylinker with more than 6 unique restriction cleavage sites.

2. A binary vector as claimed in claim 1, **characterized in that** the vector has a size of 2 to 12, preferably 3-9, especially preferably 4-6 kb.

3. A binary vector as claimed in any of claims 1-2, **characterized by** polylinkers with 15-20, especially preferably 16-25, unique restriction cleavage sites.

4. A binary vector as claimed in any of claims 1-3, **characterized by** the presence of recognition sites for sequencing primers pUC18 uni and reverse primers flanking the polylinker.

5. A binary vector as claimed in any of claims 1-4, **characterized in that** the selection marker is cloned adjacent to the left border, independently of the polylinker.

6. A binary vector as claimed in any of claims 1-5, **characterized in that** the bacterial selection marker is adjacent to the right border.

7. A binary vector as claimed in any of claims 1-6, **characterized by** increased biosafety owing to the removal of a region comprising the nic site.

8. A binary vector as claimed in any of claims 1-7, **characterized in that** it comprises the recognition motifs for recombinases flanking the insertion site for the selection markers.

9. The binary vector for the effective transformation of plant systems, comprising a T-DNA into which a heterologous nucleotide sequence is inserted and which is flanked by a right and a left border, where the rector comprises, adjacent to the left border, an additional sequence as shown in SEQ ID NO 2 or a sequence motif with at least 80%, preferably 90%, homologies to SEQ ID NO 2, and, between the borders, a polylinker with more than 6 unique restriction cleavage sites.

10. A method for generating a binary vector as claimed in any of claims 1-9, **characterized in that**
a. following deletion of the T-DNA and adjacent segments of a vector of the pPZP family, other border-comprising PCR fragments, a multicloning site and a unique site for cloning the selection markers are incorporated;
b. all NotI sites and other regions of the vector, some of which are not functional, are removed by means of two different deletions.

11. A method as claimed in claim 10, **characterized in that**, following step (b), the recognition sites for recombinases are introduced.

12. A method as claimed in claim 10 or 11, **characterized in that**, in the first step (a), the bacterial resistance marker of the vector is incorporated adjacent to the right border.

13. The use of a binary vector as claimed in any of claims 1-9 for efficiently transforming plant systems.

14. The use of a binary vector as claimed in any of claims 1-9 for generating transformed plant systems, regenerated cells or plants therefrom, and their progeny or seeds.

15. The use of a binary vector as claimed in any of claims 1-9 for establishing gene libraries.

16. The use of a binary vector as claimed in any of claims 1-9 for transforming plants, preferably mediated via agrobacteria.

## Revendications

1. Vecteur binaire pour la transformation efficace de systèmes végétaux, contenant un ADN de transfert, dans lequel une séquence nucléotidique hétérologue est insérée, et qui est flanqué d'une bordure droite et d'une bordure gauche, le vecteur comportant, au voisinage de la bordure gauche, la séquence supplémentaire selon SEQ ID N°1, et un lieur multisite entre les bordures, ayant plus de 6 sites de restriction uniques.

2. Vecteur binaire selon la revendication 1, **caractérisé en ce que** le vecteur a une taille de 2 à 12, de préférence de 3-9, d'une manière particulièrement préférée de 4-6 kb.

3. Vecteur binaire selon l'une des revendications 1-2, **caractérisé par** un lieur multisite ayant 15-20, d'une manière particulièrement préférée 16-25 sites de restriction uniques.

4. Vecteur binaire selon l'une des revendications 1-3, **caractérisé par** la présence de sites de reconnaissance de séquences amorces uni pUC18 et amorces inverses qui flanquent le lieur multisite.

5. Vecteur binaire selon l'une des revendications 1-4, **caractérisé par** le clonage, indépendant du lieur multisite, du marqueur de sélection à côté de la bordure gauche.

6. Vecteur binaire selon l'une des revendications 1-5, **caractérisé par** la position du marqueur de sélection bactérien au voisinage de la bordure droite.

7. Vecteur binaire selon l'une des revendications 1-6, **caractérisé par** une biosécurité accrue grâce à l'élimination d'une région qui contient le site nic.

8. Vecteur binaire selon les revendications 1-7, **caractérisé en ce qu'**il comprend les motifs de reconnaissance de recombinases flanquant le site d'insertion du marqueur de sélection.

9. Vecteur binaire pour la transformation efficace de systèmes végétaux, contenant un ADN de transfert dans lequel une séquence nucléotidique hétérologue est insérée et qui est flanqué d'une bordure droite et d'une bordure gauche, le vecteur comportant, au voisinage de la bordure gauche, une séquence supplémentaire contenant un motif de séquence selon SEQ ID N°2 ou un motif de séquence présentant des homologies d'au moins 80 %, de préférence de 90 % avec SEQ ID N°2, et un lieur multisite entre les bordures, ayant plus de 6 sites de restriction uniques.

10. Procédé de fabrication d'un vecteur binaire selon l'une des revendications 1-9, **caractérisé en ce que**
a. après délétion de l'ADN de transfert et des segments voisins d'un vecteur de la famille pPZP, on incorpore d'autres fragments PCR contenant les bordures, un site de multiclonage, et un site unique pour insertion par clonage des marqueurs de sélection ;
b. par 2 délétions différentes, tous les sites NotI, et d'autres domaines en partie non fonctionnels du vecteur, sont éliminés.

11. Procédé selon la revendication 10, **caractérisé en ce que**, après l'étape (b), on introduit les sites de reconnaissance de recombinases.

12. Procédé selon les revendications 10 ou 11, **caractérisé en ce que**, dans la première étape (a), on incorpore le marqueur de résistance bactérienne au voisinage de la bordure droite.

13. Utilisation d'un vecteur binaire selon l'une des revendications 1-9 pour la transformation efficace de systèmes végétaux.

14. Utilisation d'un vecteur binaire selon l'une des revendications 1-9 pour fabriquer des systèmes végétaux transformés, des cellules régénérées ou des plantes qui en proviennent, leur descendance ou leurs semences.

15. Utilisation d'un vecteur binaire selon l'une des revendications 1-9 pour réaliser des génothèques.

16. Utilisation d'un vecteur binaire selon l'une des revendications 1 à 9 pour la transformation de végétaux, médiée de préférence par des agrobactéries.
